# EUROPÄISCHE PATENTSCHRIFT

(11) **EP 3 571 182 B1**
(45) Veröffentlichungstag und Bekanntmachung des Hinweises auf die Patenterteilung: **22.06.2022**
(21) Anmeldenummer: 17822567.8
(22) Anmeldetag: 04.12.2017
(51) Int. Cl.: C07C 211/36, C08G 59/50

(54) **N,N'-DIAMINOPROPYL-2-METHYL-CYCLOHEXAN-1,3-DIAMIN UND N,N'-DIAMINOPROPYL-4-METHYL-CYCLOHEXAN-1,3-DIAMIN UND DEREN VERWENDUNG ALS HÄRTER FÜR EPOXIDHARZE**
N,N -DIAMINOPROPYL-2-METHYL-CYCLOHEXANE-1,3-DIAMINE AND N,N'-DIAMINOPROPYL-4-METHYL-CYCLOHEXANE-1,3-DIAMINE AND THEIR USE AS HARDENERS FOR EPOXY RESINS
N,N -DIAMINOPROPYL-2-MÉTHYL-CYCLOHEXANE-1,3-DIAMINE ET N,N'-DIAMINOPROPYL-4-MÉTHYL-CYCLOHEXANE-1,3-DIAMINE ET LEUR UTILISATION COMME DURCISSEURS DE RESINES EPOXYDES

(30) Priorität: 05.12.2016 EP 16202180
(43) Veröffentlichungstag der Anmeldung: 27.11.2019
(73) Patentinhaber: BASF SE, 67056 Ludwigshafen am Rhein (DE)
(72) Erfinder: GRUENANGER, Christian, 67056 Ludwigshafen (DE); PANCHENKO, Alexander, 67056 Ludwigshafen (DE); GORMAN, Irene, 67056 Ludwigshafen (DE); STEGMANN, Veit, 67056 Ludwigshafen (DE); MELDER, Johann-Peter, 67056 Ludwigshafen (DE); GUTFRUCHT, Norbert, 67056 Ludwigshafen (DE); ERNST, Martin, 67056 Ludwigshafen (DE); HOFMANN, Marc, 26123 Oldenburg (DE)
(74) Vertreter: BASF IP Association
(86) Internationale Anmeldenummer: PCT/EP2017/081313
(87) Internationale Veröffentlichungsnummer: WO 2018/104206

(56) Entgegenhaltungen:
- WO-A1-2011/032877
- WO-A1-2011/107512
- US-A- 4 321 354

## Beschreibung

Die vorliegenden Erfindung betrifft die Polyamine *N*,*N*'-Diaminopropyl-2-methyl-cyclohexan-1,3-diamin und *N*,*N*'-Diaminopropyl-4-methyl-cyclohexan-1,3-diamin bzw. deren Mischungen, deren Verwendung als Härter für Epoxidharz, sowie eine härtbare Zusammensetzung umfassend Epoxidharz und diese Polyamine. Die Erfindung betrifft weiter die Härtung dieser Zusammensetzung sowie das durch Härtung dieser Zusammensetzung erhaltene gehärtete Epoxidharz.

Epoxidharze sind allgemein bekannt und werden aufgrund ihrer Zähigkeit, Flexibilität, Haftung und chemischen Beständigkeit als Materialien zur Oberflächenbeschichtung, als Klebstoffe und zum Formen und Laminieren verwendet sowie zur Herstellung von faserverstärkten Verbundmaterialen.

Eine wichtige Anwendung von Epoxidharzen ist die Oberflächenbeschichtung und da insbesondere die Bodenbeschichtung (flooring). Für diesen Zweck werden Härter benötigt, die eine schnelle Härtung, auch bei niedrigen Temperaturen, ermöglichen. Die Beschichtung soll möglichst schnell nach dem Aufbringen auf die Oberfläche belastet werden können (Begehbarkeit von Bodenbeschichtungen), also eine ausreichend hohe Härte (z.B. Shore D Härte) aufweist. Auch eine hohe Glasübergangstemperatur der Beschichtung, sodass die Beschichtung bei hohen Nutzungstemperaturen stabil bleibt, ist ein wichtiges Kriterium. Für eine Beschichtung von der Feuchtigkeit ausgesetzten Oberflächen (bspw. Bodenbeschichtungen im Freien) ist zudem eine gute Frühwasserbeständigkeit wichtig.

Typische Härter für Epoxidharze sind Polyamine, die eine Polyadditionsreaktion (Kettenverlängerung) bewirken. Polyamine mit hoher Reaktivität werden im Allgemeinen erst kurz vor der gewünschten Härtung zugesetzt. Bei solchen Systemen handelt es sich daher um sogenannte zweikomponentige (2K) -Systeme.

Bei Bodenbeschichtungen werden insbesondere aliphatische und cycloaliphatische Polyamine als Härter für Epoxidharze verwendet (Ullmann's Encyclopedia of Industrial Chemistry, Wiley-VCH, Weinheim, Germany, 2012, Vol. 13, Epoxy Resins, H. Pham & M. Marks (online: 15.10.2005, DOI: 10.1002/14356007.a09_547.pub2)). Dabei weisen aliphatische Polyamine wie Diethylentriamin (DETA), Hexamethylendiamin (HMD) oder Triethylentetraamin (TETA), in der Regel eine hohe Reaktivität aus und ermöglichen so auch bei Raumtemperatur oder moderat erhöhter Temperatur eine schnelle Härtung. Die cycloaliphatischen Polyamine, wie bspw. Isophorondiamin (IPDA), Bis(4-Aminocyclohexyl)methan (PACM), 1,2-Diaminocyclohexan (1,2-DACH), Dimethyldicycan (DMDC), 4-Methylcyclohexan-1,3-diamin (4-MCDA), 2-Methylcyclohexan-1,3-diamin (2-MCDA) oder ein Gemisch aus 2-MCDA und 4-MCDA (MCDA), reagieren üblicherweise etwas langsamer, was in der Regel auch mit einer längeren Topfzeit einhergeht. Mit cycloaliphatischen Aminen gehärtete Epoxidharze zeichnen sich üblicherweise durch vergleichsweise gute elektrische, mechanische und thermische Eigenschaften, wie insbesondere eine vergleichsweise hohe Glasübergangstemperatur (Tg), aus.

Die Härtung von Epoxidharzen mit 1,2-DACH ermöglicht zwar hohe Glasübergangstemperaturen, aber die hohe Flüchtigkeit und die Toxizität (insbesondere die Hautsensibilisierung) machen die Verwendung dieses Amines problematisch. So wird in US 4321354 ein mit Aminopropylgruppen substituiertes Derivat des 1,2-DACH als Härter bereitgestellt, das weniger flüchtig und toxisch sei.

MCDA, das ebenfalls für die Härtung von Epoxidharzen beschrieben ist (EP-B 443344; WO 2011/032877), ist weit weniger flüchtig und toxisch als 1,2-DACH.

Wünschenswert wäre ein aminischer Härter zur Härtung von Epoxidharzen für Bodenbeschichtungsanwendungen, der die schnelle Härtung von aliphatischen Polyaminen, insbesondere bei niedrigen Temperaturen wie Raumtemperatur oder darunter oder bei moderat erhöhten Temperaturen wie 75°C, mit den guten thermischen Eigenschaften (hohe Glasübergangstemperatur) von cycloaliphatischen Polyaminen vereint. Ein solcher Härter sollte insbesondere auch im Niedertemperaturbereich einsetzbar sein und da eine schnelle Härtung ermöglichen, bei der eine vergleichsweise hohe Härte (z.B. Shore D Härte) möglichst schnell erreicht wird. Zudem ist wünschenswert, dass ein solcher Härter eine gute Frühwasserbeständigkeit aufweist.

Als der Erfindung zugrundeliegende Aufgabe kann daher erachtet werden die Bereitstellung eines solchen aminischen Härters, geeignet zur Härtung von Epoxidharzen insbesondere für Bodenbeschichtungsanwendungen, der schnelle Härtung auch bei geringen Temperaturen, gute thermische und mechanische Eigenschaften und gute Frühwasserbeständigkeit vereint.

Entsprechend betrifft die vorliegende Erfindung die Bereitstellung eines Polyamins, ausgewählt aus der Gruppe, bestehend aus *N*,*N*'-Diaminopropyl-2-methyl-cyclohexan-1,3-diamin (DAP-2-MCDA) und *N*,*N*'-Diaminopropyl-4-methyl-cyclohexan-1,3-diamin (DAP-4-MCDA). In einer besonderen Ausführungsform betrifft die vorliegende Erfindung Mischungen aus diesen Polyaminen (DAP-MCDA).

Die Molekülstruktur von DAP-2-MCDA und DAP-4-MCDA ist in den folgenden Formeln wiedergegeben:

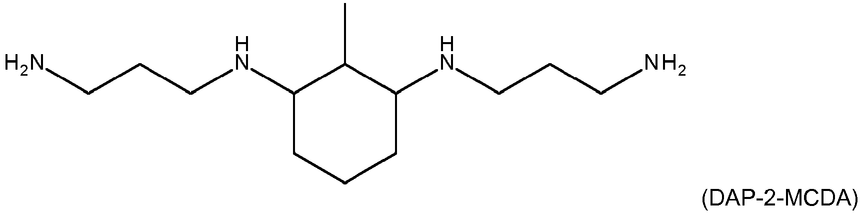

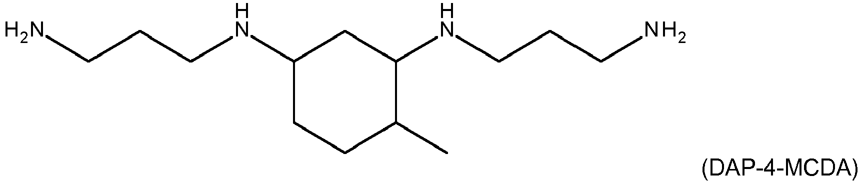

Ein weiterer Gegenstand der vorliegenden Erfindung betrifft ein Verfahren zur Herstellung von DAP-2-MCDA, DAP-4-MCDA oder DAP-MCDA, umfassend

Schritt 1, in dem 2-MCDA, 4-MCDA oder MCDA mit Acrylnitril zu dem entsprechenden cyanethylierten Zwischenprodukt umgesetzt wird, und

Schritt 2, in dem das cyanethylierte Zwischenprodukt mit Wasserstoff katalytisch zum DAP-2-MCDA, DAP-4-MCDA bzw. DAP-MCDA hydriert wird.

Das in Schritt 1 des erfindungsgemäßen Herstellungsverfahrens eingesetzte 2-MCDA, 4-MCDA oder MCDA ist erhältlich durch Kernhydrierung von 2,6-Toluoldiamin (2,6-TDA), 2,4-Toluoldiamin (2,4-TDA) bzw. eines Gemisches aus 2,4- und 2,6-TDA (EP-B 443344; WO 2011/032877).

Vorzugsweise erfolgt die Umsetzung in Schritt 1 des erfindungsgemäßen Herstellungsverfahrens bei einer Temperatur von 20 bis 100°C. In einer Variante des erfindungsgemäßen Herstellungsverfahrens wird das Acrylnitril in Schritt 1 in überstöchiometrischer Menge eingesetzt, und der Überschuss des Acrylnitrils nach der Umsetzung mit dem 2-MCDA, 4-MCDA bzw. MCDA mit einem niedermolekularen Amin wie bspw. Dimethylamin umgesetzt. Vorzugsweise wird für die Hydrierung in Schritt 2 des erfindungsgemäßen Herstellungsverfahrens ein Raney-Katalysator eingesetzt, bspw. ein Kobalt-Raney-Katalysator. Vorzugsweise erfolgt die Hydrierung in Schritt 2 des erfindungsgemäßen Herstellungsverfahrens bei einem Wasserstoffpartialdruck von 20 bis 200 bar absolut. Vorzugsweise erfolgt die Hydrierung in Schritt 2 des erfindungsgemäßen Herstellungsverfahrens bei einer Temperatur von 80 bis 150°C. In einer Variante des erfindungsgemäßen Herstellungsverfahrens wird das in Schritt 1 erhaltene cyanethylierte Zwischenprodukt vor der Weiterverwendung in Schritt 2 in einem Schritt 1a gereinigt, bspw. destillativ. Vorzugsweise wird das in Schritt 2 des erfindungsgemäßen Herstellungsverfahrens erhaltene DAP-2-MCDA, DAP-4-MCDA bzw. DAP-MCDA in einem anschließenden Schritt 2a gereinigt, bspw. destillativ.

Ein weiterer Gegenstand der vorliegenden Erfindung betrifft eine härtbare Zusammensetzung umfassend ein oder mehrere Epoxidharze und ein oder mehrere Polyamine, ausgewählt aus der Gruppe bestehend aus DAP-2-MCDA und DAP-4-MCDA.

Epoxidharze gemäß dieser Erfindung besitzen üblicherweise 2 bis 10, bevorzugt 2 bis 6, ganz besonders bevorzugt 2 bis 4 und insbesondere 2 Epoxidgruppen. Bei den Epoxidgruppen handelt es sich insbesondere um Glycidylethergruppen, wie sie bei der Umsetzung von Alkoholgruppen mit Epichlorhydrin entstehen. Bei den Epoxidharzen kann es sich um niedermolekulare Verbindungen, welche im Allgemeinen ein mittleres Molgewicht (Mₙ) kleiner 1.000 g/mol haben oder um höhermolekulare Verbindungen (Polymere) handeln. Solche polymeren Epoxidharze haben bevorzugt einen Oligomerisierungsgrad von 2 bis 25, besonders bevorzugt von 2 bis 10 Einheiten. Es kann sich um aliphatische, auch cycloaliphatische Verbindungen oder um Verbindungen mit aromatischen Gruppen handeln. Insbesondere handelt es sich bei den Epoxidharzen um Verbindungen mit zwei aromatischen oder aliphatischen 6-Ringen oder deren Oligomere. Technisch von Bedeutung sind Epoxidharze, die durch Umsetzung des Epichlorhydrins mit Verbindungen, welche mindestens zwei reaktive H-Atome haben, insbesondere mit Polyolen, erhältlich sind. Von besonderer Bedeutung sind Epoxidharze, die durch Umsetzung des Epichlorhydrins mit Verbindungen, welche mindestens zwei, vorzugsweise zwei Hydroxylgruppen und zwei aromatische oder aliphatische 6-Ringe enthalten, erhältlich sind. Als derartige Verbindungen seien insbesondere Bisphenol A und Bisphenol F, sowie hydriertes Bisphenol A und Bisphenol F genannt - die entsprechenden Epoxidharze sind die Diglycidylether von Bisphenol A oder Bisphenol F, oder hydriertem Bisphenol A oder Bisphenol F. Als Epoxidharz gemäß dieser Erfindung wird vorzugsweise ein Epoxidharz eingesetzt, das ausgewählt ist aus der Gruppe bestehend aus Diglycidylether von Bisphenol A, Diglycidylether von Bisphenol F, Diglycidylether von hydriertem Bisphenol A und Diglycidylether von hydriertem Bisphenol F. Als Epoxidharz gemäß dieser Erfindung wird üblicherweise Bisphenol-A-diglycidylether (DGEBA) verwendet. Geeignete Epoxidharze gemäß dieser Erfindung sind auch Tetraglycidyl-Methylendianilin (TGMDA) und Triglycidylaminophenol oder Gemische davon. In Betracht kommen auch Umsetzungsprodukte des Epichlorhydrins mit anderen Phenolen, z.B. mit Kresolen oder Phenol-Aldehyd-Adukten, wie Phenolformaldehydharzen, insbesondere Novolaken. Geeignet sind auch Epoxidharze, welche sich nicht vom Epichlorhydrin ableiten. In Betracht kommen z.B. Epoxidharze, welche Epoxidgruppen durch Umsetzung mit Glycidyl(meth)acrylat) enthalten. Vorzugsweise werden erfindungsgemäß Epoxidharze oder Gemische davon eingesetzt, die bei Raumtemperatur (25 °C) flüssig sind. Das Epoxid-Äquivalenzgewicht (EEW) gibt die durchschnittlich Masse des Epoxidharzes in g pro Mol Epoxidgruppe an.

Vorzugsweise besteht die erfindungsgemäße härtbare Zusammensetzung zu mindestens 50 Gew.-% aus Epoxidharz.

Die erfindungsgemäße härtbare Zusammensetzung kann ein oder mehrere Reaktivverdünner enthalten. Reaktivverdünner im Sinne der Erfindung sind Verbindungen, die die Anfangsviskosität der härtbaren Zusammensetzung herabsetzen und im Verlauf der Härtung der härtbaren Zusammensetzung mit dem sich ausbildenden Netzwerk aus Epoxidharz und Härter eine chemische Bindung eingehen. Bevorzugte Reaktivverdünner im Sinne dieser Erfindung sind niedermolekulare organische, vorzugsweise aliphatische Verbindungen mit ein oder mehreren Epoxidgruppen, sowie cyclische Carbonate, insbesondere cyclische Carbonate mit 3 bis 10 C-Atomen, beispielsweise Ethylencarbonat, Propylencarbonat, Butylencarbonat oder Vinylencarbonat, sein. Erfindungsgemäße Reaktivverdünner sind vorzugsweise ausgewählt aus der Gruppe bestehend aus Ethylencarbonat, Vinylencarbonat, Propylencarbonat, 1,4-Butandiolbisglycidylether, 1,6-Hexandiolbisglycidylether (HDDE), Glycidylneodecanoat, Glycidylversatat, 2-Ethylhexylglycidylether, Neopentylglykoldiglycidylether, p-tert-Butylglycidether, Butylglycidether, C₈-C₁₀- Alkylglycidylether, C₁₂-C₁₄- Alkylglycidylether, Nonylphenylglycidether, p-tert-Butylphenylglycidether, Phenylglycidether, o-Cresylglycidether, Polyoxypropylenglycoldiglycidether, Trimethylolpropantriglycidether (TMP), Glycerintriglycidether, Triglycidylparaaminophenol (TGPAP), Divinylbenzyldioxid und Dicyclopentadiendiepoxid. Sie sind vorzugsweise ausgewählt aus der Gruppe bestehend aus 1,4-Butandiolbisglycidylether, C₈-C₁₀- Alkylmonoglycidylether, C₁₂-C₁₄- Alkylmonoglycidylether, 1,6-Hexandiolbisglycidylether (HDDE), Neopentylglykoldiglycidylether, Trimethylolpropantriglycidether (TMP), Glycerintriglycidether und Dicyclopentadiendiepoxid.

Die erfindungsgemäßen Reaktivverdünner machen vorzugsweise einen Anteil bis 30 Gew.-%, besonders bevorzugt bis 25 Gew.-%, insbesondere bis 20 Gew.-% bezogen auf die Harzkomponente (Epoxidharz und etwaig eingesetzte Reaktivverdünner) der härtbaren Zusammensetzung aus. In einer besonderen Ausführungsform machen die erfindungsgemäßen Reaktivverdünner mindestens 5 Gew.-%, insbesondere mindestens 10 Gew.-% bezogen auf die Harzkomponente (Epoxidharz und etwaig eingesetzte Reaktivverdünner) der härtbaren Zusammensetzung aus.

Die erfindungsgemäße härtbare Zusammensetzung kann neben DAP-2-MCDA, DAP-4-MCDA bzw. DAP-MCDA noch ein oder mehrere weitere aminische Härter enthalten. Vorzugsweise macht DAP-2-MCDA, DAP-4-MCDA bzw. DAP-MCDA mindestens 50 Gew.-%, besonders bevorzugt mindestens 80 Gew.-%, ganz besonders bevorzugt mindestens 90 Gew.-% bezogen auf die Gesamtmenge der aminischen Härter (DAP-2-MCDA, DAP-4-MCDA bzw. DAP-MCDA und etwaige weitere aminischen Härter) in der härtbaren Zusammensetzung aus. In einer besonderen Ausführungsform enthält die härtbare Zusammensetzung neben DAP-2-MCDA, DAP-4-MCDA bzw. DAP-MCDA keine weiteren aminischen Härter. Unter einem aminischen Härter ist im Rahmen der vorliegenden Erfindung ein Amin mit einer NH-Funktionalität von ≥ 2 zu verstehen (demnach weist bspw. ein primäres Monoamin eine NH-Funktionalität von 2, ein primäres Diamin eine NH-Funktionalität von 4 und ein Amin mit 3 sekundären Aminogruppen eine NH-Funktionalität von 3 auf).

Vorzugsweise werden bei der erfindungsgemäßen härtbaren Zusammensetzung die Epoxidharze (inklusive etwaige Reaktivverdünner mit ihren jeweiligen reaktiven Gruppen) und aminische Härter in einem bezogen auf die reaktiven Gruppen der Verbindungen der Harzkomponente (Epoxidgruppen und bspw. etwaige Carbonatgruppen) bzw. die NH-Funktionalität in etwa stöchiometrischen Verhältnis eingesetzt. Besonders geeignete Verhältnisse von reaktiven Gruppen der Verbindungen der Harzkomponente zu NH-Funktionalität sind beispielsweise 1 : 0,8 bis 1 : 1,2. Reaktive Gruppen der Verbindungen der Harzkomponente sind solche Gruppen, die unter den Härtungsbedingungen mit den Aminogruppen des aminischen Härters chemisch reagieren.

Die erfindungsgemäße härtbare Zusammensetzung kann auch ein oder mehrere weitere Zusätze wie beispielsweise inerte Verdünner, Härtungsbeschleuniger, Verstärkungsfasern (insbesondere Glass- oder Carbonfasern), Pigmente, Farbstoffe, Füllstoffe, Trennmittel, Zähigkeit erhöhende Agenzien (toughener), Fließmittel, schaumhemmende Agenzien (anti-foamer), flammhemmende Agenzien oder Eindickungsmittel. Solche Zusätze werden üblicherweise in funktioneller Menge zugegeben, also bspw. ein Pigment in einer Menge, die zu der gewünschten Farbe für die Zusammensetzung führt. Üblicherweise enthalten die erfindungsgemäßen Zusammensetzungen von 0 bis 50 Gew.-%, bevorzugt 0 bis 20 Gew.-%, bspw. 2 bis 20 Gew.-% für die Gesamtheit aller Additive bezogen auf die gesamte härtbare Zusammensetzung. Unter Additive werden im Rahmen dieser Erfindung alle Zusätze zur härtbaren Zusammensetzung verstanden, die weder Epoxidverbindung noch Reaktivverdünner noch aminischer Härter sind.

Ein weiterer Gegenstand der Erfindung ist ein Verfahren zur Herstellung von gehärteten Epoxidharzen aus der erfindungsgemäßen härtbaren Zusammensetzung. Bei diesem Verfahren wird die erfindungsgemäße härtbare Zusammensetzung bereitgestellt und anschließend gehärtet. Dazu werden die Komponenten (Epoxidharz und Härter (umfassend DAP-2-MCDA und/oder DAP-4-MCDA) und gegebenenfalls weitere Komponenten wie beispielsweise Reaktivverdünner, Reaktionsbeschleuniger oder sonstige Additive) miteinander in Kontakt gebracht, vermischt und danach bei einer für die Anwendung praktikablen Temperatur gehärtet. Bevorzugt erfolgt die Härtung bei einer Temperatur von mindestens 0 °C, besonders bevorzugt von mindestens 10 °C.

Ein besonderer Gegenstand der Erfindung ist ein Verfahren zur Herstellung von Beschichtungen, insbesondere Bodenbeschichtungen, dadurch gekennzeichnet, dass die erfindungsgemäße härtbare Zusammensetzung bereitgestellt, auf eine Oberfläche aufgetragen und anschließend gehärtet wird. Dazu werden die Komponenten (Epoxidharz, Härter (umfassend DAP-2-MCDA und/oder DAP-4-MCDA) und gegebenenfalls weitere Komponenten wie beispielsweise Reaktivverdünner, Reaktionsbeschleuniger oder sonstige Additive) miteinander in Kontakt gebracht, vermischt, auf eine Oberfläche aufgetragen und danach bei einer für die Anwendung praktikablen Temperatur gehärtet. Bevorzugt erfolgt die Härtung bei einer Temperatur von mindestens 0 °C, besonders bevorzugt von mindestens 10 °C.

Vorzugsweise wird das gehärtete Epoxidharz noch einer thermischen Nachbehandlung ausgesetzt, beispielsweise im Rahmen der Härtung oder im Rahmen einer optionalen nachgeschalteten Temperung.

Die Härtung kann bei Normaldruck und bei Temperaturen kleiner 250°C, insbesondere bei Temperaturen kleiner 185°C, vorzugsweise bei Temperaturen kleiner 100°C erfolgen, insbesondere in einem Temperaturbereich von 0 bis 185°C, ganz besonders bevorzugt in einem Temperaturbereich von 10 bis 130°C, ganz besonders bevorzugt in einem Temperaturbereich von 10 bis 75°C, insbesondere in einem Temperaturbereich von 10 bis 35°C.

Weiterer Gegenstand der Erfindung ist das gehärtete Epoxidharz aus der erfindungsgemäßen härtbaren Zusammensetzung. Insbesondere ist gehärtetes Epoxidharz, das erhältlich ist, bzw. erhalten wird durch Härtung einer erfindungsgemäßen härtbaren Zusammensetzung, ein Gegenstand der Erfindung. Insbesondere ist gehärtetes Epoxidharz, das erhältlich ist, bzw. erhalten wird durch das erfindungsgemäße Verfahren zur Herstellung von gehärteten Epoxidharzen, ein Gegenstand der Erfindung.

Die erfindungsgemäßen härtbaren Zusammensetzungen eignen sich als Beschichtungsmittel oder Imprägnierungsmittel, als Klebstoff, zur Herstellung von Formkörpern und Verbundmaterialien, oder als Gießmassen zur Einbettung, Anbindung oder Verfestigung von Formkörpern. Als Beschichtungsmittel seien z.B. Lacke genannt. Insbesondere können mit den erfindungsgemäßen härtbaren Zusammensetzungen kratzfeste Schutzlacke auf beliebigen Substraten, z.B. aus Metall, Kunststoff oder Holzwerkstoffen erhalten werden. Die härtbaren Zusammensetzungen eignen sich auch als Isolierbeschichtungen in elektronischen Anwendungen, z.B. als Isolierbeschichtung für Drähte und Kabel. Genannt sei auch die Verwendung zur Herstellung von Photoresisten. Sie eignen sich auch als Reparaturlack, z.B. auch bei der Ausbesserung von Rohren ohne Demontage der Rohre (cure in place pipe (CIPP) rehabilitation).

Die erfindungsgemäßen härtbaren Zusammensetzungen eignen aufgrund ihrer Frühwasserbeständigkeit für die Härtung in Gegenwart von Wasser oder Luftfeuchte. Gegenstand dieser Erfindung ist daher auch ein Verfahren zur Herstellung von gehärteten Epoxidharzen aus der erfindungsgemäßen härtbaren Zusammensetzung, wobei die Härtung in Gegenwart von Wasser oder Luftfeuchte, insbesondere von Luftfeuchte, insbesondere mit einer relativen Luftfeuchte von mindestens 30%, ganz besonders mit einer relativen Luftfeuchte von mindestens 50% erfolgt.

Bei Härtung im Niedertemperaturbereich (beispielsweise 0 bis 30°C) vereinen die erfindungsgemäßen härtbaren Zusammensetzungen eine vergleichsweise hohe Shore D Härte, die auch vergleichsweise schnell erreicht wird, mit einer vergleichsweise kurzen Gelierzeit. Gleichzeitig weisen sie eine sehr gute Frühwasserbeständigkeit auf. Mit diesem Eigenschaftsprofil sind die erfindungsgemäßen härtbaren Zusammensetzungen in besonderer Weise geeignet für Bodenbeschichtungen.

Ein weiterer Gegenstand der vorliegenden Erfindung betrifft die Verwendung eines oder mehrerer Polyamine, ausgewählt aus der Gruppe bestehend aus DAP-2-MCDA und DAP-4-MCDA, als Härter für Epoxidharze, insbesondere als Härter für die Herstellung von Epoxidharz-basierten Beschichtungen, insbesondere Bodenbeschichtungen (flooring).

Unter dem Begriff Raumtemperatur ist im Rahmen der vorliegenden Erfindung die Temperatur von 23°C zu verstehen.

Die Glasübergangstemperatur (Tg) kann mittels Dynamisch-Mechanischer Analyse (DMA), beispielsweise gemäß der Norm DIN EN ISO 6721, oder mit einem Differential Kalorimeter (differential scanning calorimetry; DSC), beispielsweise gemäß der Norm DIN 53765, bestimmt werden. Bei der DMA wird ein rechteckiger Probekörper mit einer erzwungenen Frequenz und vorgegebener Deformation auf Torsion belastet. Dabei wird die Temperatur mit einer definierten Rampe gesteigert und Speicher- und Verlustmodul in festen Zeitintervallen aufgezeichnet. Ersterer stellt die Steifigkeit eines viskoelastischen Werkstoffs dar. Letzterer ist proportional zur im Material dissipierten Arbeit. Die Phasenverschiebung zwischen der dynamischen Spannung und der dynamischen Verformung wird durch den Phasenwinkel δ gekennzeichnet. Die Glasübergangstemperatur kann durch unterschiedliche Methoden bestimmt werden: Als Maximum der tan-δ Kurve, als Maximum des Verlustmoduls oder mittels Tangentenmethode am Speichermodul. Bei Bestimmung der Glasübergangstemperatur unter Verwendung eines Differential Kalorimeters wird eine sehr kleine Probenmenge (ca. 10 mg) in einem Aluminiumtiegel erwärmt und der Wärmestrom zu einem Referenztiegel gemessen. Dieser Zyklus wird wiederholt und die Bestimmung des Glasübergangs erfolgt beim zweiten Zyklus. Die Auswertung der Tg-Stufe der Wärmestromkurve kann über den Wendepunkt, nach der halben Breite oder dem Verfahren der Mittelpunktstemperatur bestimmt werden.

Die Gelierzeit gibt nach DIN 16 945 einen Anhaltspunkt über die Zeitspanne zwischen der Zugabe des Härters zum Reaktionsgemisch und des Übergangs der Reaktionsharzmasse vom flüssigen in den Gelzustand. Die Temperatur spielt dabei eine wichtige Rolle, weshalb die Gelierzeit jeweils für eine vorbestimmte Temperatur ermittelt wird. Mit Hilfe dynamisch-mechanischer Methoden, insbesondere der Rotationsviskosimetrie, können auch kleine Probenmengen quasi-isotherm untersucht und ihr gesamter Viskositäts- bzw. Steifigkeitsverlauf erfasst werden. Nach der Norm ASTM D 4473 ist der Schnittpunkt zwischen dem Speichermodul G' und dem Verlustmodul G", an dem die Dämpfung tan-δ den Wert 1 hat, der Gelpunkt, und die Zeitspanne ab Zugabe des Härters zum Reaktionsgemisch bis zum Erreichen des Gelpunkts ist die Gelierzeit. Die so bestimmte Gelierzeit kann als Maß für die Aushärtungszeit angesehen werden.

Die Shore-Härte ist eine Kennzahl für Polymere wie bspw. gehärtete Epoxidharze, die in direkter Beziehung zur Eindringtiefe eines Eindringkörpers (Indenter) in den Prüfkörper steht, und ist somit ein Maß für die Härte des Prüfkörpers. Sie wird bspw. entsprechend der Norm DIN ISO 7619-1 bestimmt. Man unterscheidet zwischen den Verfahren Shore A, C und D. Als Indenter wird ein federbelasteter Stift aus gehärtetem Stahl verwendet. Dabei wird der Indenter mit Federkraft in den Prüfkörper gedrückt und die Eindringtiefe stellt ein Maß für die Shore-Härte dar. Während für die Bestimmung der Shore-Härte A und C wird als Indenter ein Kegelstumpf mit einer Stirnfläche von 0,79 mm im Durchmesser und einem Öffnungswinkel von 35° verwendet wird, benutzt man bei der Shore-Härte D-Prüfung als Indenter ein Kegelstumpf mit einer kugelförmigen Spitze mit einem Radius von 0,1 mm und einem Öffnungswinkel von 30°. Für die Ermittlung der Shore-Härtekennwerte wurde eine Skala eingeführt, die von 0 Shore (2,5 mm Eindringtiefe) bis 100 Shore (0 mm Eindringtiefe) reicht. Dabei entspricht der Skalenwert 0 dem maximal möglichen Eindruck, d.h. der Werkstoff setzt dem Eindringen des Indenters keinen Widerstand entgegen. Dagegen entspricht der Skalenwert 100 einem sehr hohen Widerstand des Werkstoffs gegenüber dem Eindringen und es wird praktisch kein Eindruck erzeugt.

Bei der Bestimmung der Shore-Härte spielt die Temperatur eine entscheidende Rolle, so dass die Messungen in einem eingeschränkten Temperaturintervall von beispielsweise 23°C ± 2 °C normgerecht durchgeführt werden müssen. Bei Bodenbeschichtungen wird üblicherweise davon ausgegangen, dass ab einer Shore-D-Härte von 45 der Boden wieder begehbar ist.

Frühwasserbeständigkeit ist die Eigenschaft einer Beschichtung, schon nach kurzer Zeit nach dem Auftragen mit Wasser oder Luftfeuchte in Kontakt kommen zu können, ohne dass die Beschichtung Schaden nimmt. Bei Beschichtungen auf Basis von Epoxidharzen und aminischen Härtern handelt es sich dabei insbesondere um Carbamatbildung, die an der Ausbildung von weißen Schlieren oder Krusten auf der Oberfläche der frischen Beschichtung zu erkennen ist ("blushing" und "blooming").

Abbildungen:

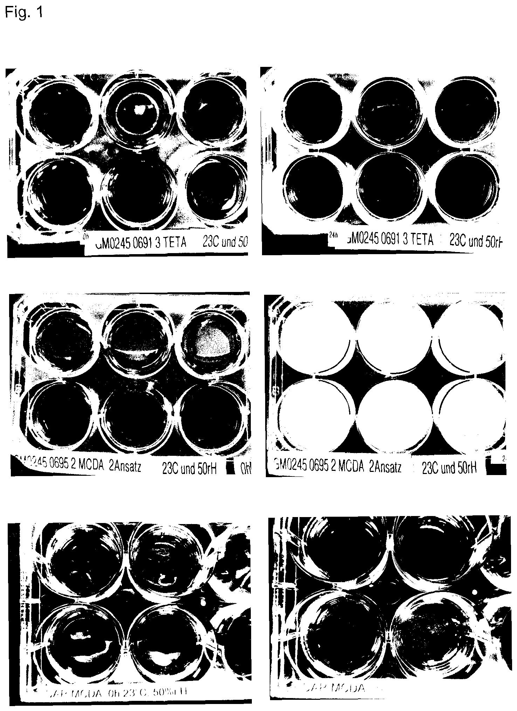

Fig. 1 zeigt das Ergebnis der Carbamatbildung gemäß Beispiel 5 für die Härter TETA (obere Bilder), MCDA (mittlere Bilder) und DAP-MCDA (untere Bilder), jeweils zu Beginn des Versuchs (links) und nach 24-stündiger Inkubation bei Raumtemperatur und 50% relativer Luftfeuchte (rechts). Deutlich zu erkennen ist die weiße Trübung, die sich nach der Inkubation in den Testschalen für MCDA gebildet hat. Im Unterschied dazu blieben die Ansätze mit TETA und DAP-MCDA ungetrübt.

### Beispiele

### Beispiel 1:

### Herstellung von DAP-MCDA

572 g (4,5 mol) MCDA (Baxxodur^{®} EC210, BASF), ein Isomerengemisch aus 4-MCDA und 2-MCDA, hergestellt durch Kernhydrierung eines Gemisches aus 2,6-Toluoldiamin (15-25%) und 2,4-Toluoldiamin (75-85%) an einem suspendierten Ruthenium Katalysator bei 230°C und 230 bar Wasserstoff-Druck, wurden zusammen mit Wasser (76,2 g) in einem Rührgefäß vorgelegt. Acrylnitril (474,9 g; 9,0 mol) wurde bei 26°C über einen Zeitraum von 7 h zugegeben, wobei sich das Reaktionsgemisch dabei leicht auf 31°C erwärmte. Das Reaktionsgemisch wurde über einen Zeitraum von 12 h bei 60°C gerührt. Nachdem noch kein Vollumsatz festgestellt wurde, wurde weiteres Acrylnitril (45 g; 0,9 mol) bei 60°C zugegeben und noch weitere 16 h gerührt. Überschüssiges Acrylnitril wurde mit einer wässrigen Dimethylaminlösung (40%ig; 95,5 g, 0,85 mol) bei 60°C abreagiert. Aus dem Reaktionsgemisch wurden die Leichtsieder destillativ bei 3,5 mbar und 200°C Sumpftemperatur entfernt und das so erhaltene bis-cyanethylierte Zwischenprodukt in der nachfolgenden Hydrierung eingesetzt.

50 g des bis-cyanethylierten Zwischenprodukts wurden in einen Rührautoklaven überführt. Als Hydrier-Katalysator wurde ein Raney Kobalt Katalysator (5 g) eingesetzt. Als Lösungsmittel wurde Tetrahydrofuran (75 g) verwendet und als Additiv wurde Natriumhydroxid (50%ig in Wasser; 0,1 g) zugesetzt. Der Autoklav wurde verschlossen und zwei Mal mit Stickstoff gespült. Anschließend wurde auf 120°C aufgeheizt und mit Wasserstoff ein Druck von 100 bar aufgepresst. Die Hydrierung wurde über einen Zeitraum von 5 h durchgeführt und der Autoklav anschließend abgekühlt und entspannt. Der Katalysator wurde abfiltriert und das Lösungsmittel THF am Rotationsverdampfer entfernt. Mehrere Ansätze wurden vereinigt und das Produkt (DAP-MCDA) wurde destillativ aufgereinigt. Aus 232 g Rohmaterial wurden 129 g des Zielproduktes mit einer Reinheit von > 98% (Analyse per GC-Flächen%) erhalten.

### Beispiel 2:

### Härtung von Epoxidharz mit DAP-MCDA

DAP-MCDA aus Beispiel 1 und Epoxidharz (Bisphenol-A-Diglycidylether, Epilox^{®} A19-03, Leuna, EEW: 184 g/mol) wurden im stöchiometrischen Verhältnis in einem Rührwerk gemischt (1min bei 2000 rpm). Unmittelbar im Anschluss auf die Vermischung wurden DSC-Messungen (differential scanning calorimetry) und rheologische Untersuchungen durchgeführt. Zum Vergleich wurden auch entsprechende Zusammensetzungen mit TETA (Akzo-Nobel) bzw. MCDA (BASF,) in gleicher Weise untersucht. Die DSC-Untersuchungen der Härtungsreaktion von DAP-MCDA bzw. TETA oder MCDA zur Bestimmung von Onset-Temperatur (To), Maximaltemperatur (Tmax), exothermer Energie (ΔH) und Glasübergangstemperaturen (Tg) wurden nach ASTM D 3418 durchgeführt, wobei das folgende Temperaturprofil verwendet wurde: 0 °C → 5K/min 180 °C → 30min 180 °C → 20K/min 0 °C → 20K/min 220 °C. Für den 2ten Lauf wurde das folgende Temperaturprofil verwendet: 0 °C → 20K/min 220 °C. Die Tg wurde beim 2ten Lauf bestimmt. Die Ergebnisse sind in Tabelle 1 zusammengestellt.

Die rheologischen Messungen zur Untersuchung des Reaktivitätsprofils der verschiedenen aminischen Härter (TETA, MCDA und DAP-MCDA) mit dem Epoxidharz wurden an einem schubspannungsgesteuerten Platte-Platte Rheometer (MCR 301, Anton Paar) mit einem Plattendurchmesser von 15 mm und einem Spaltabstand von 0,25 mm bei unterschiedlichen Temperaturen durchgeführt. Bestimmt wurde die Zeit (Topfzeit, als Maß für die Zeitspanne, in der die Reaktionsharzmasse handhabbar ist), die die frisch hergestellten Reaktionsharzmasse benötigte um eine Viskosität von 10.000 mPa*s bei einer definierten Temperatur zu erreichen. Die Messung wurde rotierend an dem oben genannten Rheometer bei verschiedenen Temperaturen (10 °C, 23 °C bzw. 75 °C) durchgeführt. Gleichzeitig wurde die Anfangsviskosität (gemittelt über den Zeitraum von 2 bis 5 min nach dem Vermischen der Komponenten) für die jeweiligen Mischungen bei den jeweiligen Temperaturen bestimmt. Schließlich wurden die Gelierzeiten bestimmt. Diese Messungen wurden oszillierend an dem oben genannten Rheometer bei 10 °C, 23 °C bzw. 75 °C durchgeführt. Der Schnittpunkt von Verlustmodul (G") und Speichermodul (G') liefert die Gelierzeit. Die Ergebnisse der rheologischen Messungen sind in Tabelle 2 zusammengefasst.

**Tabelle 1: Exothermie-Profil und Glasübergangstemperatur für die Härtung von Epoxidharz mit DAP-MCDA bzw. zum Vergleich mit TETA oder MCDA**

| | To (°C) | Tmax (°C) | ΔH (J/g) | Tg (°C) |
|---|---|---|---|---|
| TETA | 62 | 90 | 570 | 141 |
| MCDA | 78 | 108 | 528 | 169 |
| DAP-MCDA | 68 | 98 | 540 | 152 |

**Tabelle 2: Rheologisches Profil für die Härtung von Epoxidharz mit DAP-MCDA bzw. zum Vergleich mit TETA oder MCDA**

| | | Anfangsviskosität (mPa*s) | Topfzeit (min) | Gelierzeit (min) |
|---|---|---|---|---|
| 10°C | TETA | 9931 | n.a. | 693 |
| | MCDA | 6096 | 83 | 1830 |
| | DAP-MCDA | 18573 | n.a. | 910 |
| 23°C | TETA | 2098 | 58 | 282 |
| | MCDA | 1290 | 160 | 727 |
| | DAP-MCDA | 3170 | 58 | 365 |
| 75°C | TETA | 88 | 7 | 11 |
| | MCDA | 56 | 20 | 52 |
| | DAP-MCDA | 197 | 9 | 15 |

| | | | | |
|---|---|---|---|---|
| n.a.: nicht anwendbar | | | | |

Die Gelierzeit als Maß für die Härtungszeit unterscheidet sich für die eingesetzten Härter (DAP-MCDA und zum Vergleich TETA oder MCDA) deutlich. DAP-MCDA weist eine im Vergleich zu MCDA deutlich reduzierte Topfzeit und Gelierzeit auf. Die bei der DAP-MCDA-Härtung erreichte Glasübergangstemperatur ist deutlich höher als die bei der TETA-Härtung.

### Beispiel 3:

### Mechanische Eigenschaften des mit DAP-MCDA gehärteten Epoxidharzes

DAP-MCDA aus Beispiel 1 und Epoxidharz (Bisphenol-A-Diglycidylether, Epilox^{®} A19-03, Leuna, EEW: 184 g/mol) wurden im stöchiometrischen Verhältnis in einem Rührwerk gemischt (1min bei 2000 rpm) und anschließen bei erhöhter Temperatur (2 h 80°C, 3 h 125°C) gehärtet. Die mechanischen Parameter (Elastitätsmodul (tensile moduls; E-t), Zugfestigkeit (tensile strength; σ-M), Zugdehnung (tesile elongation; *έ* -M), Biegemodul (flexural modulus; E-f), Biegefestigkeit (flexural strength; σ-fM), Biegeverlängerung (flexural elongation; έ-fM)) wurden gemäß ISO 527-2:1993 und ISO 178:2006 bestimmt. Zum Vergleich wurden auch entsprechende Zusammensetzungen mit TETA (TETA, Akzo-Nobel) bzw. MCDA (Baxxodur^{®} EC210, BASF) in gleicher Weise untersucht.

**Tabelle 3: Mechanische Eigenschaften von mit DAP-MCDA gehärteten Epoxidharz im Vergleich zu TETA oder MCDA gehärtetem Epoxidharz**

| | Dehnungsversuch | | | Biegeversuch | | |
|---|---|---|---|---|---|---|
| | E-t (MPa) | σ-M (MPa) | έ-M (%) | E-f (MPa) | σ-fM (MPa) | έ -fM (%) |
| TETA | 2882 | 73 | 7 | 2991 | 110 | 6 |
| MCDA | 2894 | 86 | 7 | 2889 | 125 | 6 |
| DAP-MCDA | 2704 | 72 | 5 | 2790 | 105 | 6 |

Die Werte für Elastizitätsmodul, Biegemodul, Zugfestigkeit und Biegefestigkeit sind für das mit DAP-MCDA gehärtete Epoxidharz etwas geringer als für das mit MCDA oder das mit TETA gehärtete Epoxidharz.

### Beispiel 4:

### Shore D Härte des mit DAP-MCDA gehärteten Epoxidharzes

DAP-MCDA aus Beispiel 1 und Epoxidharz (Bisphenol-A-Diglycidylether, Epilox^{®} A19-03, Leuna, EEW: 184 g/mol) wurden im stöchiometrischen Verhältnis in einem Rührwerk gemischt (1min bei 2000 rpm) und anschließen bei Raumtemperatur (23 °C) bzw. bei 10 °C (Klimaschrank bei 65% relative Luftfeuchte) über einen Zeitverlauf von 8 Tagen gehärtet. Die Shore D Härte wurde während dieser Zeit (nach 1, 2, 3 und 8 Tagen) gemäß DIN ISO 7619-1 an den Probenkörpern (Dicke 35-36 mm) mittels Durometer (TI Shore Prüfstand, Sauter Messtechnik) bestimmt. Zum Vergleich wurden auch entsprechende Zusammensetzungen mit TETA (TETA, Akzo-Nobel) bzw. MCDA (Baxxodur^{®} EC210, BASF) in gleicher Weise untersucht.

**Tabelle 4: Shore D Härte von mit DAP-MCDA gehärteten Epoxidharz im Vergleich zu TETA oder MCDA gehärtetem Epoxidharz**

| | bei Raumtemperatur | | | | | | | | |
|---|---|---|---|---|---|---|---|---|---|
| | 5 h | 6h | 8h | 9 h | 10 h | 1 Tag | 2 Tage | 3 Tage | 8 Tage |
| TETA | 80 | 84 | 84 | 84 | 84 | 86 | 86 | 86 | 86 |
| MCDA | n.b. | n.b. | 64 | 79 | 84 | 84 | 84 | 84 | 85 |
| DAP-MCDA | n.b. | 76 | 83 | 85 | 86 | 86 | 87 | 87 | 88 |

| | bei 10°C | | | | | | | | |
|---|---|---|---|---|---|---|---|---|---|
| | | | | | | 1 Tag | 2 Tage | 3 Tage | 8 Tage |
| TETA | | | | | | 75 | 83 | 84 | 85 |
| MCDA | | | | | | n.b. | 69 | 78 | 79 |
| DAP-MCDA | | | | | | 82 | 82 | 83 | 84 |

| | | | | | | | | | |
|---|---|---|---|---|---|---|---|---|---|
| n.b.: nicht bestimmbar | | | | | | | | | |

DAP-MCDA gehärtetes Harz entwickelt im Vergleich zu MCDA deutlich schneller eine hohe Shore D Härte, etwa vergleichbar mit TETA gehärtetem Harz. Dabei wird die hohe Shore D Härte selbst bei niedrigen Temperaturen wie 10°C auch sehr schnell erreicht.

### Beispiel 5:

### Carbamatbildung

DAP-MCDA und zum Vergleich TETA und MCDA wurden in Schalen gefüllt und in einem Klimaschrank bei einer Temperatur von 23°C und einer relativen Luftfeuchtigkeit von 50% inkubiert. Nach 24 h wurde die Bildung von Carbamat (weißlicher Niederschlag) visuell überprüft. Während bei MCDA eine deutliche Carbamat-Bildung zu beobachten war, konnten bei DAP-MCDA und TETA keine Anzeichen für eine Carbamat-Bildung festgestellt werden (Fig. 1). Cabamat-Bildung ist ein Beleg unzureichender Frühwasserbeständigkeit. Somit weißen DAP-MCDA und TETA im Unterschied zu MCDA eine sehr gute Frühwasserbeständigkeit auf.

## Patentansprüche

1. Polyamin, ausgewählt aus der Gruppe, bestehend aus N,N'-Diaminopropyl-2-methylcyclohexan-1,3-diamin und N,N'-Diaminopropyl-4-methyl-cyclohexan-1,3-diamin.

2. Verfahren zur Herstellung des Polyamins gemäß Anspruch 1 oder einer Mischung der Polyamine gemäß Anspruch 1, umfassend
Schritt 1, in dem 2-Methyl-cyclohexan-1,3-diamin, 4-Methyl-cyclohexan-1,3-diamin bzw. eine Mischung aus 2-Methyl-cyclohexan-1,3-diamin und 4-Methyl-cyclohexan-1,3-diamin mit Acrylnitril zu dem entsprechenden cyanethylierten Zwischenprodukt umgesetzt wird, und
Schritt 2, in dem das cyanethylierte Zwischenprodukt mit Wasserstoff katalytisch zum N,N'-Diaminopropyl-2-methyl-cyclohexan-1,3-diamin, zum N,N'-Diaminopropyl-4-methyl-cyclohexan-1,3-diamin bzw. zu einer Mischung aus N'-Diaminopropyl-2-methyl-cyclohexan-1,3-diamin und N,N'-Diaminopropyl-4-methyl-cyclohexan-1,3-diamin hydriert wird.

3. Das Verfahren nach Anspruch 2, **dadurch gekennzeichnet, dass** das in Schritt 1 erhaltene cyanethylierten Zwischenprodukt vor der Weiterverwendung in Schritt 2 in einem Zwischenschritt 1a gereinigt wird.

4. Das Verfahren nach Anspruch 2 oder 3, **dadurch gekennzeichnet, dass** das in Schritt 2 erhaltene N,N'-Diaminopropyl-2-methyl-cyclohexan-1,3-diamin, N,N'-Diaminopropyl-4-methyl-cyclohexan-1,3-diamin bzw. die Mischung aus N'-Diaminopropyl-2-methyl-cyclohexan-1,3-diamin und *N*,*N*'-Diaminopropyl-4-methyl-cyclohexan-1,3-diamin in einem anschließenden Schritt 2a gereinigt wird.

5. Das Verfahren nach einem der Ansprüche 2 bis 4, **dadurch gekennzeichnet, dass** für die Hydrierung in Schritt 2 ein Raney-Katalysator eingesetzt wird.

6. Härtbare Zusammensetzung umfassend ein oder mehrere Epoxidharze und ein oder mehrere Polyamine gemäß Anspruch 1.

7. Die härtbare Zusammensetzung nach Anspruch 6, **dadurch gekennzeichnet, dass** die ein oder mehreren Epoxidharze ausgewählt sind aus der Gruppe, bestehend aus Diglycidylether von Bisphenol A, Diglycidylether von Bisphenol F, Diglycidylether von hydriertem Bisphenol A und Diglycidylether von hydriertem Bisphenol F.

8. Die härtbare Zusammensetzung nach Anspruch 6 oder 7, **dadurch gekennzeichnet, dass** sie ein oder mehrere Reaktivverdünner enthält.

9. Die härtbare Zusammensetzung nach einem der Ansprüche 6 bis 8, **dadurch gekennzeichnet, dass** sie neben den Polyaminen gemäß Anspruch 1 noch ein oder mehrere weitere aminische Härter enthält.

10. Die härtbare Zusammensetzung nach einem der Ansprüche 6 bis 9, **dadurch gekennzeichnet, dass** sie noch ein oder mehrere weitere Zusätze enthält.

11. Verfahren zur Herstellung von gehärteten Epoxidharzen, **dadurch gekennzeichnet, dass** eine härtbare Zusammensetzung gemäß einem der Ansprüche 6 bis 10 bereitgestellt und anschließend gehärtet wird.

12. Das Verfahren nach Anspruch 11, **dadurch gekennzeichnet, dass** die Härtung in Gegenwart von Luftfeuchte erfolgt.

13. Gehärtetes Epoxidharz, **dadurch gekennzeichnet, dass** es erhältlich ist durch das Verfahren gemäß Anspruch 11.

14. Gehärtetes Epoxidharz, **dadurch gekennzeichnet, dass** es erhältlich ist durch Härtung einer härtbaren Zusammensetzung gemäß einem der Ansprüche 6 bis 10.

15. Verwendung eines oder mehrerer Polyamine gemäß Anspruch 1 als Härter für Epoxidharze für Bodenbeschichtungen.

## Claims

1. A polyamine selected from the group consisting of N,N'-diaminopropyl-2-methyl-cyclohexane-1,3-diamine and N,N'-diaminopropyl-4-methylcyclohexane-1,3-diamine.

2. A process for producing the polyamine according to claim 1 or a mixture of the polyamines according to claim 1, comprising
step 1 in which 22-methyl-cyclohexane-1,3-diamine, 4-methyl-cyclohexane-1,3-diamine or a mixture of 2-methyl-cyclohexane-1,3-diamine and 4-methyl-cyclohexane-1,3-diamine is reacted with acrylonitrile to afford the corresponding cyanoethylated intermediate and
step 2 in which the cyanoethylated intermediate is subjected to catalytic hydrogenation with hydrogen to afford N,N'-diaminopropyl-2-methyl-cyclohexane-1,3-diamine, N,N'-diaminopropyl-4-methyl-cyclohexane-1,3-diamine or a mixture of N'-diaminopropyl-2-methyl-cyclohexane-1,3-diamine and N,N'-diaminopropyl-4-methyl-cyclohexane-1,3-diamine.

3. The process according to claim 2, wherein the cyanoethylated intermediate obtained in step 1 is purified in an intermediate step 1a prior to further use in step 2.

4. The process according to claim 2 or 3, wherein the N,N'-diaminopropyl-2-methyl-cyclohexane-1,3-diamine, N,N'-diaminopropyl-4-methyl-cyclohexane-1,3-diamine or the mixture of N'-diaminopropyl-2-methyl-cyclohexane-1,3-diamine and N,N'-diaminopropyl-4-methyl-cyclohexane-1,3-diamine obtained in step 2 is purified in a subsequent step 2a.

5. The process according to any of claims 2 to 4, wherein a Raney catalyst is used for the hydrogenation in step 2.

6. A curable composition comprising one or more epoxy resins and one or more polyamines according to claim 1.

7. The curable composition according to claim 6, wherein the one or more epoxy resins are selected from the group consisting of diglycidyl ether of bisphenol A, diglycidyl ether of bisphenol F, diglycidyl ether of hydrogenated bisphenol A and diglycidyl ether of hydrogenated bisphenol F.

8. The curable composition according to claim 6 or 7, wherein said composition comprises one or more reactive diluents.

9. The curable composition according to any of claims 6 to 8, wherein in addition to the polyamines according to claim 1 said composition also comprises one or more further aminic curing agents.

10. The curable composition according to any of claims 6 to 9, wherein said composition also comprises one or more further additions.

11. A process for producing cured epoxy resins, wherein a curable composition according to any of claims 6 to 10 is provided and subsequently cured.

12. The process according to claim 11, wherein the curing is carried out in the presence of atmospheric humidity.

13. A cured epoxy resin, wherein said resin is obtainable by the process according to claim 11.

14. A cured epoxy resin, wherein said resin is obtainable by curing a curable composition according to any of claims 6 to 10.

15. The use of one or more polyamines according to claim 1 as a curing agent for epoxy resins for floor coatings.

## Revendications

1. Polyamine, choisie dans le groupe constitué par la N,N'-diaminopropyl-2-méthyl-cyclohexane-1,3-diamine et la N,N'-diaminopropyl-4-méthyl-cyclohexane-1,3-diamine.

2. Procédé pour la préparation de la polyamine selon la revendication 1 ou d'un mélange des polyamines selon la revendication 1, comprenant
une étape 1, dans laquelle de la 2-méthyl-cyclohexane-1,3-diamine, de la 4-méthyl-cyclohexane-1,3-diamine ou un mélange de 2-méthyl-cyclohexane-1,3-diamine et de 4-méthyl-cyclohexane-1,3-diamine est transformé(e) avec de l'acrylonitrile pour donner le produit intermédiaire cyanoéthylé correspondant, et
une étape 2, dans laquelle le produit intermédiaire cyanoéthylé est hydrogéné de manière catalytique avec de l'hydrogène pour donner la N,N'-diaminopropyl-2-méthyl-cyclohexane-1,3-diamine, la N,N'-diaminopropyl-4-méthyl-cyclohexane-1,3-diamine ou un mélange de la N'-diaminopropyl-2-méthyl-cyclohexane-1,3-diamine et de la N,N'-diaminopropyl-4-méthyl-cyclohexane-1,3-diamine.

3. Procédé selon la revendication 2, **caractérisé en ce que** le produit intermédiaire cyanoéthylé obtenu dans l'étape 1 est purifié dans une étape intermédiaire la avant l'utilisation ultérieure dans l'étape 2.

4. Procédé selon la revendication 2 ou 3, **caractérisé en ce que** la N,N'-diaminopropyl-2-méthyl-cyclohexane-1,3-diamine, la N,N'-diaminopropyl-4-méthyl-cyclohexane-1,3-diamine ou le mélange de la N'-diaminopropyl-2-méthyl-cyclohexane-1,3-diamine et de N,N'-diaminopropyl-4-méthyl-cyclohexane-1,3-diamine obtenu(e) dans l'étape 2 est purifié(e) dans une étape consécutive 2a.

5. Procédé selon l'une quelconque des revendications 2 à 4, **caractérisé en ce qu'**un catalyseur de Raney est utilisé pour l'hydrogénation dans l'étape 2.

6. Composition durcissable comprenant une ou plusieurs résines époxy et une ou plusieurs polyamines selon la revendication 1.

7. Composition durcissable selon la revendication 6, **caractérisée en ce que** la ou les résines époxy sont choisies dans le groupe constitué par l'éther de diglycidyle de bisphénol A, l'éther de diglycidyle de bisphénol F, l'éther de diglycidyle de bisphénol A hydrogéné et l'éther de diglycidyle de bisphénol F hydrogéné.

8. Composition durcissable selon la revendication 6 ou 7, **caractérisée en ce qu'**elle contient un ou plusieurs diluants réactifs.

9. Composition durcissable selon l'une quelconque des revendications 6 à 8, **caractérisée en ce qu'**elle contient, outre les polyamines selon la revendication 1, encore un ou plusieurs agents de durcissement aminiques supplémentaires.

10. Composition durcissable selon l'une quelconque des revendications 6 à 9, **caractérisée en ce qu'**elle contient encore un ou plusieurs additifs supplémentaires.

11. Procédé pour la préparation de résines époxy durcies, **caractérisé en ce qu'**une composition durcissable selon l'une quelconque des revendications 6 à 10 est mise à disposition et ensuite durcie.

12. Procédé selon la revendication 11, **caractérisé en ce que** le durcissement est réalisé en présence d'humidité de l'air.

13. Résine époxy durcie, **caractérisée en ce qu'**elle peut être obtenue par le procédé selon la revendication 11.

14. Résine époxy durcie, **caractérisée en ce qu'**elle peut être obtenue par durcissement d'une composition durcissable selon l'une quelconque des revendications 6 à 10.

15. Utilisation d'une ou plusieurs polyamines selon la revendication 1 en tant qu'agent de durcissement pour des résines époxy pour des revêtements de sol.
